# EUROPEAN PATENT SPECIFICATION

(11) **EP 2 699 225 B1**
(45) Date of publication and mention of the grant of the patent: **18.12.2019**
(21) Application number: 12720635.7
(22) Date of filing: 17.04.2012
(51) Int. Cl.: A61K 8/67, A61Q 19/00, A61K 8/49, A61K 8/31

(54) **COMBINATION OF CAROTENOID, PHYTOOESTROGEN AND VITAMIN C FOR MOISTURIZING THE SKIN**
VERWENDUNG VON EINER KOMBINATION EINES KAROTENOID, EINES PHYTOÖSTROGEN UND VITAMIN C ALS WIRKSTOFF ZUR FEUCHTIGKEITSVERSORGUNG DER HAUT.
UTILISATION DE LA COMBINAISON D'UN CAROTÉNOÏDE, D'UN HYTOESTROGÉNE ET DE VITAMINE C COMME PRINCIPE ACTIF POUR L'HYDRATATION DE LA PEAU

(30) Priority: 19.04.2011 FR 1153396; 21.06.2011 US 201161499198 P
(43) Date of publication of application: 26.02.2014
(73) Proprietor: NUTRICOS Technologies, 92117 Clichy Cedex (FR)
(72) Inventor: PICCARDI, Nathalie, F-21310 Arceau (FR); BRU, Carole, F-75014 Paris (FR)
(74) Representative: Nony
(86) International application number: PCT/IB2012/051925
(87) International publication number: WO 2012/143857

(56) References cited:
- WO-A1-2009/050101
- DE-U1-202004 013 660
- FR-A1- 2 885 491
- FR-A1- 2 939 040
- CHOI ET AL: "Cosmeceuticals", SEMINARS IN CUTANEOUS MEDICINE AND SURGERY, W.B. SAUNDERS, PHILADELPHIA, US, vol. 25, no. 3, 1 September 2006 (2006-09-01), pages 163-168, XP005703032, ISSN: 1085-5629, DOI: 10.1016/J.SDER.2006.06.010

## Description

The present invention relates to the cosmetic field of moisturizing the skin.

The skin, a barrier for protection against and exchange with the environment, is both resistant and fragile.

It is known that the *stratum corneum* or horny layer, which is the outermost region of the epidermis, is most particularly involved in moisturizing the skin. Located at the interface with the external environment, its function is especially to delay excessive water loss arising from the deeper layers of the epidermis. The *stratum corneum* also protects against mechanical attack and the passage of chemical products and foreign microorganisms. It also constitutes the first line of defence against UV radiation.

With a thickness of 10 µm, it is composed of vertically stacked corneocytes surrounded by a matrix of lipid-enriched membranes. Thus, it is a two-compartment system that may be compared to a wall of bricks, composed of anuclear cells (the "bricks") and of intercellular lamellar membranes (the "cement"). By virtue of this compact stratified structure, the *stratum corneum* performs its barrier function by opposing transcutaneous water loss. It thus efficiently contributes towards the moisturization of the skin via its capacity to take up and retain water, which is mainly located in the intercellular spaces.

The skin is moisturized by the water of the deep layers and by sweat. In particular, over time, the skin loses its suppleness and its capacity to retain water decreases, then leading to dryness of the skin, which is particularly exacerbated after the age of 65.

Dry skin is characterized by a lack of water and/or a deficiency of the constituent lipids of the barrier function and/or of the hydrolipid film of the skin and/or mucous membranes. In physiological terms, dry skin is often associated with a decrease in the degree of skin moisturization and also a modification of the process of maturation of the *stratum corneum,* the most visible sign of which is the appearance of squamae at the surface of the skin. In sensory terms, dry skin may be characterized by a sensation of tautness and/or skin tension.

The dry state of the skin may have several causes:
- external factors, such as (i) sunlight, wind, cold, (ii) hard water (T° > 35°C) or chlorinated water, (iii) frequent and repeated contact of the skin with surfactants, (iv) chemical products (occupational exposure) such as solvents,
- physiological factors, such as (i) lipid-deficient skin (deficient individuals with low sebaceous secretion), (ii) elderly individuals, (iii) menopause, (iv) the newborn, (v) individuals with sensitive skin,
- iatrogenic factors, such as medicaments (retinoids, puvatherapy, lithium, dermocorticoids, etc.),
- pathological factors, such as (i) ichthyosis, (ii) atopy, (iii) psoriasis, (iv) hyperkeratosis, sensitive, reactive skin, dandruff.

Similarly, and as stated above, skin moisturization disorders, and especially skin dryness, are often observed with age. However, such states may also be manifested in young people.

Skin dryness may thus be of acquired or non-pathological constitutional origin, or it may have a pathological constitutional origin.

In the case of non-pathological constitutional dry skin, the severity of the dryness may depend on external factors. Included in this skin category are senile skin (characterized by a general decrease in skin metabolism with age), fragile skin (very sensitive to external factors and often accompanied by erythema and rosacea) and common xerosis (of probable genetic origin and manifesting mainly on the face, the limbs and the back of the hands).

In the case of acquired dry skin, the intervention of external parameters such as exposure to chemical agents, inclement climatic conditions, sunlight or certain therapeutic treatments (for example retinoids) is a determining factor. Under these external influences, the epidermis may then become momentarily and locally dry. This may concern any type of epidermis.

Irrespective of its origin, skin suffering from dryness may generally present the following signs: a rough, scaly appearance and decreased suppleness and elasticity.

Dry skin, also known as "xerosis", may appear at any age, and may be unrelated to a pathological condition. In this case, it will be referred to as "acquired" dryness.

However, xerosis becomes more frequent and troublesome with age, especially for women. It is then referred to as senile xerosis. Moreover, women generally suffer a worsening of skin dryness during the menopause, probably due to the characteristic hormonal deregulation of this phenomenon. The areas most affected are the inside of the legs, the back of the forearms and the hands.

Usually, the topical products recommended in the treatment of dry skin have three types of function: fixing water (natural moisturizing factor, humectant), retaining water (film-forming agents), reconstructing the skin barrier. The key constituent exogenous lipids of the intercellular cement and of sebum (squalene, ceramides, fatty acids) and/or active principles such as vitamin C, which are capable of stimulating the endogenous synthesis of epidermal lipids (concepts of active relipidation).

For obvious reasons, it is important to ensure a sufficient level of skin moisturization in order to preserve its suppleness, softness, tonicity and/or appearance.

There is thus a need for novel active agents in the field of skin moisturization, which are alternative or improved relative to the known active agents.

Unexpectedly, it is shown according to the invention that a combination (i) of at least one carotenoid, preferably lycopene, (ii) of at least one phytooestrogen, preferably at least one isoflavonoid, and (iii) of vitamin C, in particular in specific respective amounts, constitutes an efficient active agent for moisturizing the skin, when the said combination is administered orally.

The present disclosure describes the use of a combination (i) of at least one carotenoid, preferably lycopene, (ii) of at least one phytooestrogen, preferably an isoflavonoid, and (iii) of vitamin C, as an active agent for moisturizing the skin, for the preparation of a cosmetic composition that is suitable for orally administering a daily dose of from 1 to 25 mg of the said carotenoid, from 10 to 300 mg of the said phytooestrogen and from 10 to 1000 mg of vitamin C.

In one preferred embodiment, the active agent used in the invention consists in a combination consisting in (i) of at least one carotenoid, (ii) of at least one phytooestrogen, and (iii) of vitamin C. Therefore, in such embodiment the possible other components used are auxiliary agents without active effect on the indications considered in the invention.

The present invention relates to a cosmetic process for moisturizing the skin, comprising a step of orally administering a cosmetic composition comprising an active agent consisting in a combination (i) of at least one carotenoid, preferably lycopene, (ii) of at least one phytooestrogen, preferably an isoflavonoid, and (iii) of vitamin C, in a daily dose of from 1 to 25 mg of the said carotenoid, from 10 to 300 mg of the said isoflavonoid and from 10 to 1000 mg of vitamin C.

According to the invention, the term "carotenoid" means either a carotenoid with provitamin A activity or a carotenoid with no provitamin A activity.

Needless to say, according to the invention, the carotenoid may be a mixture of carotenoids with provitamin A activity and of carotenoids with no provitamin A activity. This mixture may be in any proportion.

According to the invention, the carotenoid with provitamin A activity may be a mixture of carotenoids with provitamin A activity. This mixture may be in any proportion.

Among the carotenoids with provitamin A activity, examples that may be mentioned include β-carotene and α-carotene.

According to the invention, the carotenoid with no provitamin A activity may be a mixture of carotenoids with no provitamin A activity. This mixture may be in any proportion. Among the carotenoids with no provitamin A activity, examples that may be mentioned include zeaxanthin, cryptoxanthin, lutein and lycopene.

The carotenoid used according to the invention may be of natural or synthetic origin. The term "natural origin" refers to a carotenoid, in pure form or in solution irrespective of its concentration in the said solution, obtained from a natural element.

According to a preferential mode of the invention, a lycopene-rich extract is used, for instance a tomato extract.

The term "synthetic origin" refers to a carotenoid, in pure form or in solution irrespective of its concentration in the said solution, obtained via chemical synthesis.

When the carotenoid is of natural origin, it may be obtained from a plant material derived from the whole plant cultured *in vivo* or derived from *in vitro* cultivation.

The term *"in vivo* cultivation" means any cultivation of standard type, i.e. in soil in the open air or in a greenhouse, or alternatively out of the soil.

The term *"in vitro* cultivation" means all the techniques known to those skilled in the art for artificially obtaining a plant or a plant part. The selection pressure imposed by the physicochemical conditions during the growth of plant cells *in vitro* makes it possible to obtain a standardized plant material that is available throughout the year, in contrast with plants cultivated *in vivo.*

Preferentially, according to the invention, a plant derived from *in vivo* cultivation is used.

Any extraction method known to those skilled in the art may be used to prepare the carotenoid used according to the invention.

The carotenoid may be in alcoholic solution, especially ethanolic solution.

The carotenoid may also be in lipidic (oil) or lipoalcoholic solution.

The carotenoids that are preferred according to the invention are β-carotene and lycopene.

Lycopene is most preferentially used.

Needless to say, if a person skilled in the art uses the carotenoid in the form of a solution or a plant extract, for example, he knows how to adjust the solution used in his composition in order for the final amount of carotenoid in the composition to be in accordance with the predefined usable amounts.

Lycopene is a natural pigment found in ripe fruit, particularly in tomatoes. It belongs to the carotenoid family and its structure is similar to that of β-carotene. Lycopene is used in compositions with tanning activity for its role in melanin synthesis (WO 97/47278), in compositions for treating the hair and/or acne for its activity on 5a-reductases (JP-2940964) or as a free-radical scavenger (JP-A-8-283136). Lycopene may be in cis or trans chemical form, and may be of natural or synthetic origin. Any process for the manufacture and/or formulation and/or encapsulation of lycopene may also be used according to the invention. Thus, examples of more complex ingredients that may be mentioned include a primary composition comprising lycopene and a whey protein. This primary composition is especially described in document WO 01/91588. This primary composition is also known as lactolycopene.

Vitamin C is commonly used in food supplements for its antioxidant activities, and is recognized by the EFSA (European Food Safety Authority) as contributing towards protecting cells from oxidative damage and contributing towards normal collagen formation and normal skin formation (EFSA Journal 2009: 7(9): 1226 [28 pp.]). Vitamin C may be in the form of ascorbic acid and derivatives thereof (esters, salts, etc.).

Phytooestrogens are a family of non-stearoyl compounds naturally produced by plants, which includes isoflavones, and also the phytooestrogens contained in hop, or lignans, in particular those of flax and of *Schizandra chinensis.*

Isoflavones are natural substances (heterocyclic phenols) belonging to the flavonoid family. In soybean grains, the concentrations of isoflavones (daidzein and genistein) or glycosylated forms thereof (daidzine, genistine) are large. By virtue of their structural similarity to female hormones of oestrogen type, genistein and daidzein are also known as phytooestrogens or phytohormones. In contrast with flavonoids, they are present in only a limited number of plants: soybean (milk, grain, meal) is the main source. As a result of these characteristics, soybean isoflavones are used as food supplements mainly for preventing or slowing down the symptoms of the menopause (Messina, 2010). Isoflavones have also been described for their antioxidant and depigmenting properties (DE-44 32 947).

The prior art discloses the use of a combination of at least one carotenoid with provitamin A activity and at least one carotenoid with no provitamin A activity for treating the signs of ageing (WO 02/34232). The use of a combination of at least one carotenoid and at least one isoflavonoid for treating the signs of ageing of the skin is also known (WO 02/34233).

FR 2 885 491 teaches compositions for oral or parenteral absorption aimed at preventing and/or treating dry and/or fragilized keratinous material which comprise one or more medium chain glycerides and at least one other agent which is active with regards to the keratinous material, and WO 2009/050101 relates to an edible product comprising encapsulated omega-3 fatty acids and/or omega-6 fatty acids and/or esters thereof with a coating comprising isoflavones aimed at promoting skin care and/or skin condition by ingestion.

However, to the Applicant's knowledge, the use of a combination of at least one carotenoid, of at least one phytooestrogen and of vitamin C for moisturizing the skin has never been described in the prior art.

Preferentially, the said at least one carotenoid is lycopene.

Preferentially, the said at least one phytooestrogen is an isoflavonoid.

For the purposes of the present invention, the term "preventing" means significantly reducing the risk of manifestation of the phenomenon under consideration.

The oral administration of active agents according to the invention has the advantage of acting globally on the skin as a whole, via a rapid mode of administration that imposes few constraints, when compared with the topical administration of active agents.

Furthermore, without wishing to be bound by any theory, the Applicant thinks that, by definition, the oral administration of active agents enables these active agents to reach physiological targets that cannot be accessed by topically administered active agents. In other words, a given combination of active agents is capable of having different activities, depending on whether this combination is administered locally (for example topically) or systemically (for example orally).

The combination (i) of at least one carotenoid, preferably lycopene, (ii) of at least one phytooestrogen, preferably an isoflavonoid, and (iii) of vitamin C above, at the indicated doses, may also be referred to as the "active moisturizer of the invention" in the present description.

It has been shown according to the invention that the combination (i) of at least one carotenoid, preferably lycopene, (ii) of at least one phytooestrogen, preferably an isoflavonoid, and (iii) of vitamin C above, when administered orally at the specified daily doses, makes it possible to maintain the water content of the skin, or to increase the water content of the skin.

The examples below illustrate the results of clinical tests performed on a group of healthy individuals, which show that the combination (i) of at least one carotenoid, preferably lycopene, (ii) of at least one phytooestrogen, preferably an isoflavonoid, and (iii) of vitamin C, when it is administered orally at the daily doses described above, especially induces a significant increase in the water content of the skin.

The water content of the skin may be measured with any type of analytical device suitable for measuring water content. Use may be made, for example, of the SHP88 "skin analyser" device.

Furthermore, it has been shown that the oral use of the moisturizing active agent of the invention, at the daily doses specified in the present description, does not simultaneously induce any adverse effects, as is illustrated especially by the absence of effects on the various physiological functions tested, especially by means of urine analyses, faecal analyses and biochemical blood analyses. The results of the examples show especially that the oral use of the moisturizing active agent of the invention, at the specified daily doses, does not induce any change in the renal and hepatic functions.

In other words, it has been shown that the moisturizing active agent for use in the process of the invention makes it possible to maintain the moisturization of the skin or to restore the moisturization of a dry skin, and is totally harmless to the user.

According to certain aspects, since the moisturizing active agent of the invention makes it possible to maintain the moisturization of the skin, it is useful for individuals who envisage being exposed to climatic conditions that are unfavourable to the skin, for example during the winter season, or, conversely, during the summer season.

Thus, the use of a combination (i) of at least one carotenoid, preferably lycopene, (ii) of at least one phytooestrogen, preferably an isoflavonoid, and (iii) of vitamin C, may especially make it possible to maintain normal skin moisturization and/or to prevent skin dryness.

In addition, the use of a combination (i) of at least one carotenoid, preferably lycopene, (ii) of at least one phytooestrogen, preferably an isoflavonoid, and (iii) of vitamin C, may especially make it possible to cosmetically treat skin dryness.

The use of the combination of the invention may also make it possible to prevent and/or treat non-pathological constitutional dryness or non-pathological acquired dryness of the skin.

The use of a combination (i) of at least one carotenoid, preferably lycopene, (ii) of at least one phytooestrogen, preferably an isoflavonoid, and (iii) of vitamin C, for moisturizing the skin, for the preparation of a cosmetic composition suitable for the oral administration of a daily dose of from 1 to 25 mg of the said carotenoid, from 10 to 300 mg of the said phytooestrogen and from 10 to 1000 mg of vitamin C, may also make it possible to prevent and/or treat skin dryness, including the cases where they are accompanied by squamous states and the itching and/or tautness associated with dry skin.

According to certain aspects, the use of the combination of the invention is especially useful for physiologically restoring a suitable state of moisturization of the *stratum corneum,* for improving the comfort of dry skin, for combating the dull and/or lifeless appearance of dry skin as a consequence of its drying-out, or for treating skin that has undergone a desiccating exogenous stress induced by: a chemical product, radiation peeling, or rubbing the skin with an external substance.

The use of the combination for use in the process of the invention may make it possible to impart and/or restore the skin's moisturized and healthy appearance, the said appearance being manifested by smooth, elastic, tonic or firm skin, or a luminous complexion.

It may make it possible to prevent and/or reduce the presence of squamae, roughness, crevasses, scales and/or wrinkles or fine lines associated with dry skin or skin with a moisturization deficit.

The combination may also make it possible, due to the maintenance or restoration of good moisturization, to maintain or reinforce the skin's barrier properties.

### Dry skin

As indicated previously, dry skin is essentially manifested by a sensation of tautness and/or tension. This skin also has a rough feel and appears covered with squamae. When the skin is slightly dry, these squamae are abundant but barely visible to the naked eye. They are less and less numerous, but increasingly visible to the naked eye, when this disorder worsens.

The origin of this skin dryness may be of constitutional or acquired type.

In the case of constitutional dry skin, two categories may be distinguished: pathological skins and non-pathological skins.

Pathological constitutional dry skins are essentially represented by atopic dermatitis and ichthyosis. They are virtually independent of the external conditions.

Atopic dermatitis is described as being associated with a deficit in the metabolism of the lipids of the *stratum corneum* and especially of ceramides. This pathology is in the form of a more or less chronic xerosis concerning a large surface area of the body, possibly combined with inflammatory and pruriginous outbreaks of plaques.

Ichthyoses are pathologies characterized by a genetic deficit affecting the keratinization process at different stages. They are manifested by substantial desquamation as plaques.

The pathological constitutional dry skins concerned according to the invention are more particularly dry skins or dry scalps of non-inflammatory origin.

In the case of non-pathological constitutional dry skin, the severity of the dryness may depend, for its part, on external factors. Included in this skin category are senile skin (characterized by a general decrease in skin metabolism with age), fragile skin (very sensitive to external factors and often accompanied by erythema and rosacea) and common xerosis (of probable genetic origin and manifesting mainly on the face, the limbs and the back of the hands).

In the case of acquired dry skin, the intervention of external parameters such as exposure to chemical agents, inclement climatic conditions, sunlight or certain therapeutic treatments (for example retinoids) is a determining factor. Under these external influences, the epidermis may then become momentarily and locally dry. This may concern any type of epidermis.

Thus, skin dryness may also be induced by an exogenous stress, of chemical origin, for example such as peeling, or of mechanical origin (rubbing or shaving).

It is recalled that a peeling operation conventionally consists in applying to the skin a chemical substance for the purpose of bringing about limited and controlled destruction of the epidermis and of the upper layers of the dermis, in order to improve certain disorders of the cutaneous appearance.

In parallel with peeling treatments which may be termed "chemical" with regard to the chemical products they use, a technique involving the use of ablative and non-ablative lasers has also been developed.

The first ablative lasers, produced with pulsed or scanned CO₂ lasers, have the immediate effect of vaporizing (or ablating) the epidermis and often the upper part of the dermis. A strip of the subjacent dermis is generally also the site of a thermal lesion with denaturation and contraction of collagen. During the cicatrization phase, re-epithelization takes place from the hair follicles and other appendages in addition to an upper dermal strip ("collagen remodelling").

The latest generation of lasers uses a system for transforming the laser beam into a multitude of spaced-out beams in order to produce on the skin spaced-out impacts, thus maintaining areas of unimpaired healthy skin between the affected areas.

For obvious reasons, peeling thus has an action which, although controlled, remains irritative on the surface of the epidermis and is of a nature to induce skin dryness.

The oral use of a combination (i) of at least one lycopene, (ii) of at least one isoflavonoid, and (iii) of vitamin C, as an active agent, at the daily doses specified in the description, thus proves to be most particularly effective:
- for treating states of skin dryness, squamous states, including dandruff,
- for treating dry skins,
- for treating the itching and/or tautness associated with dry skins,
- for physiologically restoring suitable moisturization of the *stratum corneum,*
- for treating hypo-seborrhoeic dry skins,
- for preventing and/or reducing the wrinkles associated with skin dryness,
- for improving the comfort of the skin and the scalp,
- for combating the dull and/or lifeless appearance of the skin as a consequence of its drying-out,
- for treating skins that have undergone desiccating exogenous stress induced by a chemical product, for instance a peeling composition, or induced by radiation peeling, or else induced mechanically especially by rubbing, for example on shaving.

The active agents present in the combination of active agents for use in the process of the invention, lycopene, isoflavonoid products and vitamin C, are well known in the prior art and are readily available, and especially commercially available.

### Lycopene

Lycopene is a natural pigment found in ripe fruit, particularly in tomatoes. It belongs to the carotenoid family and its structure is similar to that of β-carotene.

Lycopene is a carotenoid with no provitamin A activity.

The role of lycopene in the ripening of fruit is known in the prior art.

Lycopene may be in *cis* or *trans* chemical form.

### Isoflavonoids

Isoflavonoids constitute a subclass of flavonoids, formed from a 3-phenylchroman backbone which may comprise varied substituents and different oxidation levels. In contrast with flavonoids, they are present in only a very limited number of plants.

Isoflavonoids include several classes of compounds, among which mention may be made of isoflavones, isoflavanones, rotenoids, pterocarpans, isoflavanes, isoflavan-3-enes, 3-arylcoumarins, 3-aryl-4-hydroxycoumarins, coumestanes, coumaronochromones, α-methyldeoxybenzoins or 2-arylbenzofurans.

For a complete review of isoflavonoids, their analysis methods and their sources, a person skilled in the art may advantageously refer to chapter 5 "Isoflavonoids" written by P.M. Dewick in The Flavonoids, published by Harbone, pp. 125-157 (1988).

The isoflavonoids that are suitable for use in the present invention may be of natural or synthetic origin. The term "natural origin" means an isoflavonoid in pure form or in solution at various concentrations, obtained via various extraction processes from an element, generally a plant, of natural origin. The term "synthetic origin" means an isoflavonoid in pure form or in solution at various concentrations, obtained via chemical synthesis.

It is preferred to use isoflavonoids of natural origin. Among these, mention may be made of: daidzein, formononetine, cuneatine, genistein, isoprunetine and prunetine, cajanine, orobol, pratensein, santal, junipegenin A, glycitein, afrormosine, retusine, tectorigenin, irisolidone, jamaicine, and also analogues and/or metabolites thereof.

According to the present invention, among the isoflavonoids, it is preferred to use isoflavones, including the glycosylated forms and the aglycone forms. Among the isoflavones, the simplest isoflavones are preferred, among which are daidzein, daidzine, genistein, genistine, equol and mixtures thereof. Daidzein, daidzine, genistein and genistine are present in particular in the soybean extract (*Glycina max*) available from Archer Daniels Midland Company under the name Novasoy®.

Processes for preparing isoflavones are especially described in WO 95/10530, WO 95/10512, US-5 679 806, US-5 554 519, EP-812 837 and WO 97/26269.

In one combination of active agents according to the invention, the isoflavonoids are preferentially chosen from isoflavones, isoflavanones, rotenoids, pterocarpans, isoflavanes, isoflavan-3-enes, 3-arylcoumarins, 3-aryl-4-hydroxycoumarins, coumestanes, coumaronochromones, α-methyldeoxybenzoins and 2-arylbenzofurans.

In particular, the isoflavonoids may be chosen from daidzein, formononetine, cuneatine, genistein, isoprunetine and prunetine, cajanine, orobol, pratensein, santal, junipegenin A, glycitein, afrormosine, retusine, tectorigenin, irisolidone, jamaicine, and also analogues and/or metabolites thereof.

In addition, an isoflavonoid that is suitable for use in the invention may be chosen from genistine, daidzine, genistein, daidzein, glycitine, equol, formononetine, cuneatine, isoprunetine and prunetine, cajanine, orobol, pratensein, santal, junipegenin A, glycitein, afrormosine, retusine, tectorigenin, irisolidone, jamaicine, and also analogues and/or metabolites thereof.

In certain embodiments, the said isoflavonoid is an isoflavone, or a mixture of isoflavones.

In certain embodiments, the said isoflavonoid is chosen from daidzine, daidzein, genistine and genistein, or a mixture thereof.

In certain embodiments, the said isoflavonoid is chosen from daidzein and genistein, or a mixture thereof.

In certain embodiments, the said at least one isoflavone is in the form of a mixture of isoflavones.

In certain embodiments, the said at least one isoflavone is in the form of a mixture of an extract of soybean isoflavones.

By way of illustration, an extract of soybean isoflavones may be, for example, the extract of soybean (*Glycina max*) available from Archer Daniels Midland Company under the name Novasoy®.

### Cosmetic use

The compositions for use in the process of the invention may be in any galenical form normally available for oral administration.

As has already been specified previously, the combination (i) of at least one carotenoid, preferably lycopene, (ii) of at least one phytooestrogen, preferably an isoflavonoid, and (iii) of vitamin C, which is used orally, is included in a cosmetic composition that is suitable for administering a daily dose of from 1 to 25 mg of the said carotenoid, from 10 to 300 mg of the said phytooestrogen and from 10 to 1000 mg of vitamin C.

In certain embodiments, the said cosmetic composition is suitable for administering a daily dose of carotenoid, preferably of lycopene, ranging from 2 to 12 mg.

In certain embodiments, the said cosmetic composition is suitable for administering a daily dose of carotenoid, preferably of lycopene, ranging from 4 to 7 mg.

In certain embodiments, the said cosmetic composition is suitable for administering a daily dose of vitamin C ranging from 10 to 800 mg, especially from 10 to 600 mg, or even from 10 to 400 mg, and better still from 10 to 250 mg.

In certain embodiments, the said cosmetic composition is suitable for administering a daily dose of vitamin C ranging from 10 to 250 mg.

In certain embodiments, the said cosmetic composition is suitable for administering a daily dose of vitamin C ranging from 20 to 120 mg.

In certain embodiments, the said cosmetic composition is suitable for administering a daily dose of vitamin C ranging from 50 to 70 mg.

In certain embodiments, the said cosmetic composition is suitable for administering a daily dose of isoflavone(s) ranging from 16 to 100 mg.

In certain embodiments, the said cosmetic composition is suitable for administering a daily dose of isoflavone(s) ranging from 40 to 60 mg.

When a mixture of isoflavones is used in the form of a soybean extract, a daily dose of total isoflavones ranging from 16 to 100 mg comprises an amount of isoflavone aglycones (mainly daidzein and genistein) ranging from 8 to 50 mg.

When a mixture of isoflavones is used in the form of a soybean extract, a daily dose of total isoflavones ranging from 40 to 60 mg comprises an amount of isoflavone aglycones (mainly daidzein and genistein) ranging from 20 to 30 mg.

In particular embodiments, the said cosmetic composition is suitable for administering a daily dose of 6 mg of lycopene, 50 mg of soybean isoflavone extract and 60 mg of vitamin C.

From his general knowledge in the field of the galenical formulation of oral cosmetic compositions, a person skilled in the art can easily prepare cosmetic compositions that are suitable for use in the process of the invention, and in particular the dosage units of the said cosmetic compositions that are suitable for a daily administration of the combination of active agents at the specified doses.

In certain embodiments of the cosmetic composition, the daily dose of the combination (i) of at least one carotenoid, preferably lycopene, (ii) of at least one phytooestrogen, preferably an isoflavonoid, and (iii) of vitamin C, is included in a single dosage unit.

In other embodiments of the cosmetic composition, the daily dose of the combination (i) of at least one carotenoid, preferably lycopene, (ii) of at least one phytooestrogen, preferably an isoflavonoid, and (iii) of vitamin C, is included in a plurality of dosage units.

Thus, the daily dose of the combination of active agents may be contained in 2, 3, 4, 5, 6, 7, 8, 9 or 10 dosage units.

The process according to the invention may comprise a single administration.

According to another embodiment, the administration is repeated, for example 2 to 4 times daily for one day or more and generally for an extended period of at least 4 weeks, or even 4 to 15 weeks with, where appropriate, one or more periods of stoppage.

In one particular embodiment, the daily dose of the combination of active agents is included in two dosage units of the oral cosmetic composition.

In certain embodiments, a cosmetic composition is prepared in which the phytooestrogens, especially the isoflavone compounds, are present in a content ranging from 0.0001% to 50% by weight, preferentially from 0.001% to 30% by weight and preferably from 0.1% to 10% by weight relative to the total weight of the composition.

In certain embodiments, a cosmetic composition is prepared in which the carotenoid, and especially lycopene, is present in a content ranging from 0.0001% to 50% by weight, preferentially from 0.001% to 10% by weight and even more preferably in a content ranging from 0.02% to 2% by weight relative to the total weight of the composition.

In certain embodiments, a cosmetic composition is prepared in which the vitamin C is present in a content ranging from 0.0001% to 50% by weight, preferably from 0.1% to 30% by weight and even more preferably in a content ranging from 0.2% to 12% by weight relative to the total weight of the composition.

In certain embodiments, a cosmetic composition is prepared in which the phytooestrogens, preferably soybean isoflavones, are present in a content ranging from 0.1% to 10% by weight, the carotenoid, preferably lycopene, is present in a content ranging from 0.02% to 2% by weight, and the vitamin C is present in a content ranging from 0.2% to 12% by weight relative to the total weight of the composition.

For ingestion, numerous embodiments of oral compositions are possible.

Milk, yoghurt, cheese, fermented milks, milk-based fermented products, ice creams, products based on fermented cereals, milk-based powders, baby and infant formulations, food products of candy type, chocolate, cereals, animal feed and in particular pet food, tablets, gel capsules or soft capsules, oral supplements in dry form and oral supplements in liquid form are especially suitable for use as pharmaceutical or food supports.

For ingestion, numerous embodiments of oral compositions and especially of food supplements are possible. They are formulated via the usual processes for producing coated tablets, gel capsules, gels, emulsions, tablets, capsules, soft capsules, drinkable vials and drinks. In particular, the active agent(s) according to the invention may be incorporated in any other form of food supplements or enriched foods, for example food bars, or compacted or non-compacted powders. The powders may be diluted with water, in soda, dairy products or soybean derivatives, or may be incorporated into food bars.

The active agents according to the invention may be formulated with the usual excipients and components for such oral compositions or food supplements, i.e. especially fatty and/or aqueous components, humectants, thickeners, texture agents, taste agents and/or coating agents, antioxidants, preserving agents and dyes that are common in the food sector.

The formulating agents and excipients for oral compositions, and especially for food supplements, are known in this field and are not the subject of a detailed description herein.

A cosmetic composition for use in the process of the invention may be in the form of dosage units chosen from a drinkable solution, a syrup, a tablet, a coated tablet, a gel capsule and a capsule.

In the cosmetic composition for use in the process of the invention, the support may be of diverse nature depending on the type of composition under consideration.

In the present case of a combination for use in the process of the invention, i.e. orally, the use of an ingestible support is preferred.

Needless to say, the oral use of the combination (i) of at least one carotenoid, preferably lycopene, (ii) of at least one phytooestrogen, preferably an isoflavonoid, and (iii) of vitamin C, at the specified daily doses, may be performed using suitable oral compositions, which may also contain several additional active agents, other than the carotenoid, the phytooestrogens and vitamin C.

As active agents that may be used, mention may be made of vitamins B3, B5, B6, B8, E, D and PP, curcuminoids, sugars, amino acids, sulfureous amino acids, polyunsaturated fatty acids, probiotics, phytosterols, polyphenol extracts, and minerals such as zinc, calcium or magnesium.

In particular, use may be made of an antioxidant complex comprising selenium and vitamin E, flavonoids such as catechins, hesperidin, proanthocyanidins and anthocyanins, ubiquinones, resveratrol, coffee extracts, probiotics, prebiotics, taurine, amino acids, glutathione precursors and 3- and 6-polyunsaturated fatty acids.

### Cosmetic process

The present disclosure describes a cosmetic process for moisturizing the skin, comprising a step of orally administering a cosmetic composition comprising a combination (i) of at least one carotenoid, preferably lycopene, (ii) of at least one phytooestrogen, preferably an isoflavonoid, and (iii) of vitamin C, as an active agent, the said cosmetic composition being suitable for administering a daily dose of from 1 to 25 mg of the said carotenoid, from 10 to 300 mg of the said isoflavonoid and from 10 to 1000 mg of vitamin C.

The cosmetic process according to the invention may thus be performed by oral daily administration of the cosmetic composition described above.

The process according to the invention may comprise a single daily administration of the said cosmetic composition.

According to another embodiment, the administration is repeated, for example 2 to 3 times daily over a day.

In certain embodiments, the cosmetic process is applied over a prolonged period of at least 30 successive days, or even 4 to 15 weeks, with, where appropriate, one or more periods of interruption.

A cosmetic process for moisturizing the skin of the invention may involve the combined administration of a combination of the invention and of at least a second composition intended to have a beneficial effect on the skin.

This second composition may be administered orally or topically to the skin, and preferably topically. A second composition that is suitable for use in the invention may be any composition comprising at least one active agent, in particular a cosmetic agent, known to have a beneficial effect on the skin. Preferably, a second composition may comprise at least one cosmetic active agent that is capable of exerting a beneficial effect on moisturizing the skin. Such active agents are known to those skilled in the art.

The invention covers a process comprising the combined, simultaneous, sequential or separate administration orally of a combination of the invention, and orally or topically to the skin, preferably topically, of at least a second composition comprising an active agent, preferably a cosmetic agent, which is suitable for exerting a beneficial effect on the skin.

The examples below are presented as non-limiting illustrations of the field of the invention.

### EXAMPLES

### Examples of oral compositions

### Example 1: formulation of coated tablet type

| **Active material** | **mg/coated tablet** |
|---|---|
| Lycopene (via lactolycopene 2%) | 2 |
| Ascorbic acid | 20 |
| Soybean isoflavones (via Novasoy 400) | 16 |
| | |

| **Excipient for the core of the coated tablet** | |
|---|---|
| Encompress™ | 53 |
| Avicel PH 101 | 53 |
| Aerosil 200 | 4 |
| AcDiSol | 3 |
| Magnesium stearate | 0.5 |
| | |
| **Sugar-coating excipients** (about 85% of the mass of the core) | 242 |

| | |
|---|---|
| **This type of coated tablet may be taken 1 to 3 times a day.** | |

### Example 2: formulation of tablet type

| **Active material** | **mg/tablet** |
|---|---|
| Lycopene (via beadlets 5%) | 3 |
| Ascorbic acid | 30 |
| Soybean isoflavones (via Novasoy 400) | 25 |
| | |

| **Excipient of the tablet core** | |
|---|---|
| Dibasic calcium phosphate dihydrate | 245.80 |
| Microcrystalline Cellulose, silicified 2% | 163.87 |
| Croscarmellose sodium | 21.00 |

| **Active material** | **mg/tablet** |
|---|---|
| Silicon dioxide | 7.20 |
| Colloidal silicon dioxide | 3.50 |
| Masnesium stearate | 2.80 |
| | |

| **Coating agent** | |
|---|---|
| Opadry white 20A28380 | 21.00 |
| Purified water | qs |
| Opadry Pink 20A240005 | 18 |
| Purified water | qs |

| | |
|---|---|
| This type of tablet may be taken 1 to 2 times a day. | |

### Example of demonstration of the activity

### Example 1: Effects of a cosmetic composition comprising a combination of lycopene, a mixture of isoflavonoids and vitamin C

### 1. Cosmetic composition used

A composition in accordance with Example 1 was used.

This composition is suitable for the administration of a combination of lycopene, a mixture of isoflavones extracted from soybean and vitamin C at a daily dose of 6 mg of lycopene, 50 mg of isoflavones and 60 mg of vitamin C.

### 2. Subjects and Methods

### 2.1 Subjects

A group of 120 healthy female volunteers 30 to 50 years old, having a skin water content of less than 12, were included in this study.

The subjects were not taking any medical treatment or functional food or cosmetic composition capable of preserving the water content of the skin, throughout the period of the test. During the test, the individuals did not change their eating habits.

### 2.2 Efficacy parameters

The measurement of the skin water content was performed in a well-ventilated room, under stable temperature and humidity conditions. The measurement was taken on the forehead, between the eyebrows, cleaned beforehand with distilled water using a pad of cotton wool, at a moment when the subject was calm. The skin water content was determined 15 minutes after cleaning and drying the part of the forehead. The determination of the parameters, before and after the test, was performed with the same apparatus and by the same person.

### 3. Results

### 3.1 General conditions:

Among the 120 subjects selected, only 100 subjects effectively participated in the study, and were divided at random either into the control group (n = 50) or into the treated group (n = 50).

### 3.2 Results of the skin tests

**Table 1: effect of the cosmetic composition on the water content (+/- SD)**

| Group | Case | Water content | |
|---|---|---|---|
| | | Before taking the composition | After taking the composition |
| Treated | 50 | 8.50±2.09 | 10.88±3.23** |
| Control | 50 | 8.30±2.31 | 9.14±3.47 # |

| | | | |
|---|---|---|---|
| ** comparison of the data within the same group before and after the test, P < 0.01; after #, comparison of the data between the treated group and the control group, P < 0.05. | | | |

In the light of the results of Table 1, the skin water content in the treated group was improved, and a significant difference was shown (i) within a group, in the treated group (p < 0.01) and (ii) between groups, between the treated group and the control group at the end of the 30 days of supplementation (P < 0.05).

No significant difference was demonstrated in the control group.

In other words, the skin water content was improved in the treated group in comparison with the control group.

### 4. Conclusion

The subjects of the treated group orally absorbed the cosmetic composition for 30 successive days. A significant difference in skin water content was shown when the treated group was compared before and after the test, and also when the treated group and the control group were compared after the test, which indicates that the cosmetic composition makes it possible to increase the water content of the skin, which is consequently better hydrated.

## Claims

1. A cosmetic process for moisturizing the skin, comprising a step of orally administering a cosmetic composition comprising an active agent consisting in a combination (i) of at least one carotenoid, preferably lycopene, (ii) of at least one phytooestrogen, preferably an isoflavonoid, and (iii) of vitamin C, in a daily dose of from 1 to 25 mg of the said carotenoid, from 10 to 300 mg of the said isoflavonoid and from 10 to 1000 mg of vitamin C.

2. Cosmetic process according to claim 1, **characterized in that** it is performed by oral daily administration of the cosmetic composition.

3. Cosmetic process according to claims 1 or 2, **characterized in that** it comprises a single daily administration of the cosmetic composition.

4. Cosmetic process according to one of claims 1 to 3, wherein the administration is repeated 2 to 3 times daily over a day.

5. Cosmetic process according to one of claims 1 to 4, wherein the cosmetic process is applied over a prolonged period of at least 30 days or from 4 to 15 weeks, with optionally one or more periods of interruption.

6. Cosmetic process according to one of claims 1 to 5, **characterized in that** the said isoflavonoid is chosen from isoflavones, isoflavanones, rotenoids, pterocarpans, isoflavanes, isoflavan-3-enes, 3-arylcoumarins, 3-aryl-4-hydroxycoumarins, coumestanes, coumaronochromones, α-methyldeoxybenzoins and 2-arylbenzofurans.

7. Cosmetic process according to one of claims 1 to 6, **characterized in that** the said isoflavonoid is chosen from genistine, daidzine, genistein, daidzein, glycitine, equol, formononetine, cuneatine, isoprunetine and prunetine, cajanine, orobol, pratensein, santal, junipegenin A, glycitein, afrormosine, retusine, tectorigenin, irisolidone, jamaicine, and also analogues and/or metabolites thereof.

8. Cosmetic process according to any one of claims 1 to 6, **characterized in that** the said at least one isoflavonoid is an isoflavone, or a mixture of isoflavones.

9. Cosmetic process according to claim 7, **characterized in that** the said isoflavonoid is chosen from daidzine, daidzein, genistine and genistein, or a mixture thereof.

10. Cosmetic process according to claim 8, **characterized in that** the said at least one isoflavone is a mixture of isoflavones.

11. Cosmetic process according to claim 10, **characterized in that** the said at least one isoflavone is in the form of an extract of soybean isoflavones.

12. Cosmetic process according to one of claims 1 to 11, **characterized in that** the cosmetic composition is in the form of dosage units chosen from a drinkable solution, a syrup, a tablet, a coated tablet, a gel capsule and a capsule.

13. Cosmetic process according to one of claims 1 to 12, **characterized in that** the phytooestrogens, preferably the isoflavone compounds, are present in the cosmetic composition in a content ranging from 0.0001% to 50% by weight, preferentially from 0.001% to 30% by weight and preferably from 0.1% to 10% by weight relative to the total weight of the cosmetic composition.

14. Cosmetic process according to one of claims 1 to 13, **characterized in that** the carotenoid, preferably lycopene, is present in the cosmetic composition in a content ranging from 0.0001% to 50% by weight, preferentially from 0.001% to 10% by weight and even more preferably in a content ranging from 0.02% to 2% by weight relative to the total weight of the cosmetic composition.

15. Cosmetic process according to one of claims 1 to 14, **characterized in that** the vitamin C is present in the cosmetic composition in a content ranging from 0.0001% to 50% by weight, preferably from 0.1% to 30% by weight and even more preferably in a content ranging from 0.2% to 12% by weight relative to the total weight of the cosmetic composition.

16. Cosmetic process according to one of claims 1 to 15, **characterized in that** it further comprises the combined administration of at least a second compositions intended to have a beneficial effect on the skin.

17. Cosmetic process according to claim 16, wherein the second composition is administered orally or topically to the skin, in particular topically.

18. Cosmetic process according to claim 16, wherein the second composition comprises at least one active agent, in particular a cosmetic agent, more particularly a cosmetic active agent that is capable of exerting a beneficial effect on moisturizing the skin.

## Patentansprüche

1. Kosmetisches Verfahren zum Befeuchten der Haut, umfassend einen Schritt der oralen Verabreichung einer kosmetischen Zusammensetzung, die einen Wirkstoff umfasst, der aus einer Kombination (i) von mindestens einem Carotinoid, vorzugsweise Lycopen, (ii) mindestens einem Phytoöstrogen, vorzugsweise einem Isoflavonoid, und (iii) Vitamin C besteht, in einer Tagesdosis von 1 bis 25 mg des Carotinoids, von 10 bis 300 mg des Isoflavonoids und von 10 bis 1000 mg Vitamin C.

2. Kosmetisches Verfahren nach Anspruch 1, **dadurch gekennzeichnet, dass** es durch orale tägliche Verabreichung der kosmetischen Zusammensetzung durchgeführt wird.

3. Kosmetisches Verfahren nach Anspruch 1 oder 2, **dadurch gekennzeichnet, dass** es eine einzige tägliche Verabreichung der kosmetischen Zusammensetzung umfasst.

4. Kosmetisches Verfahren nach einem der Ansprüche 1 bis 3, wobei die Verabreichung 2- bis 3-mal täglich über einen Tag wiederholt wird.

5. Kosmetisches Verfahren nach einem der Ansprüche 1 bis 4, wobei das kosmetische Verfahren über einen längeren Zeitraum von mindestens 30 Tagen oder von 4 bis 15 Wochen angewendet wird, gegebenenfalls mit einem oder mehreren Unterbrechungszeiträumen.

6. Kosmetisches Verfahren nach einem der Ansprüche 1 bis 5, **dadurch gekennzeichnet, dass** das Isoflavonoid aus Isoflavonen, Isoflavanonen, Rotenoiden, Pterocarpanen, Isoflavanen, Isoflavan-3-enen, 3-Arylcumarinen, 3-Aryl-4-hydroxycumarinen, Coumestanen, Cumaronochromonen, α-Methyldesoxybenzoinen und 2-Arylbenzofuranen ausgewählt ist.

7. Kosmetisches Verfahren nach einem der Ansprüche 1 bis 6, **dadurch gekennzeichnet, dass** das Isoflavonoid aus Genistin, Daidzin, Genistein, Daidzein, Glycitin, Equol, Formononetin, Cuneatin, Isoprunetin und Prunetin, Cajanin, Orobol, Pratensein, Santal, Junipegenin A, Glycitein, Afrormosin, Retusin, Tectorigenin, Irisolidon, Jamaicin, sowie Analogen und/oder Metaboliten davon ausgewählt ist.

8. Kosmetisches Verfahren nach einem der Ansprüche 1 bis 6, **dadurch gekennzeichnet, dass** es sich bei dem mindestens einen Isoflavonoid um ein Isoflavon oder eine Mischung von Isoflavonen handelt.

9. Kosmetisches Verfahren nach Anspruch 7, **dadurch gekennzeichnet, dass** das Isoflavonoid aus Daidzin, Daidzein, Genistin und Genistein oder einer Mischung davon ausgewählt ist.

10. Kosmetisches Verfahren nach Anspruch 8, **dadurch gekennzeichnet, dass** es sich bei dem mindestens einen Isoflavon um eine Mischung von Isoflavonen handelt.

11. Kosmetisches Verfahren nach Anspruch 10, **dadurch gekennzeichnet, dass** das mindestens eine Isoflavon in Form eines Extrakts von Sojabohnen-Isoflavonen vorliegt.

12. Kosmetisches Verfahren nach einem der Ansprüche 1 bis 11, **dadurch gekennzeichnet, dass** die kosmetische Zusammensetzung in Form von Dosierungseinheiten, die aus einer trinkbaren Lösung, einem Sirup, einer Tablette, einer überzogenen Tablette, einer Gelkapsel und einer Kapsel ausgewählt sind, vorliegen.

13. Kosmetisches Verfahren nach einem der Ansprüche 1 bis 12, **dadurch gekennzeichnet, dass** die Phyto-östrogene, vorzugsweise die Isoflavonverbindungen, in der kosmetischen Zusammensetzung in einem Gehalt im Bereich von 0,0001 bis 50 Gew.-%, bevorzugt von 0,001 bis 30 Gew.-% und vorzugsweise von 0,1 bis 10 Gew.-%, bezogen das Gesamtgewicht der kosmetischen Zusammensetzung, vorliegen.

14. Kosmetisches Verfahren nach einem der Ansprüche 1 bis 13, **dadurch gekennzeichnet, dass** das Carotinoid, vorzugsweise Lycopen, in der kosmetischen Zusammensetzung in einem Gehalt im Bereich von 0,0001 bis 50 Gew.-%, bevorzugt von 0,001 bis 10 Gew.-% und noch weiter bevorzugt in einem Gehalt im Bereich von 0,02 bis 2 Gew.-%, bezogen auf das Gesamtgewicht der kosmetischen Zusammensetzung, vorliegt.

15. Kosmetisches Verfahren nach einem der Ansprüche 1 bis 14, **dadurch gekennzeichnet, dass** das Vitamin C in der kosmetischen Zusammensetzung in einem Gehalt im Bereich von 0,0001 bis 50 Gew.-%, vorzugsweise von 0,1 bis 30 Gew.-% noch weiter bevorzugt in einem Gehalt im Bereich von 0,2 bis 12 Gew.-%, bezogen auf das Gesamtgewicht der kosmetischen Zusammensetzung, vorliegt.

16. Kosmetisches Verfahren nach einem der Ansprüche 1 bis 15, **dadurch gekennzeichnet, dass** es ferner die kombinierte Verabreichung von mindestens einer zweiten Zusammensetzung, die eine günstige Wirkung auf die Haut haben soll, umfasst.

17. Kosmetisches Verfahren nach Anspruch 16, wobei die zweite Zusammensetzung oral oder topisch, insbesondere topisch, an die Haut verabreicht wird.

18. Kosmetisches Verfahren nach Anspruch 16, wobei die zweite Zusammensetzung mindestens einen Wirkstoff, insbesondere einen kosmetischen Wirkstoff, spezieller einen kosmetischen Wirkstoff, der eine günstige Wirkung auf die Befeuchtung der Haut ausüben kann, umfasst.

## Revendications

1. Procédé cosmétique pour l'hydratation de la peau, comprenant une étape consistant à administrer par voie orale une composition cosmétique comprenant un principe actif constitué d'une association (i) d'au moins un caroténoïde, de préférence le lycopène, (ii) d'au moins un phytoestrogène, de préférence un isoflavonoïde, et (iii) de vitamine C, en une dose quotidienne de 1 à 25 mg dudit caroténoïde, de 10 à 300 mg dudit isoflavonoïde et de 10 à 1000 mg de vitamine C.

2. Procédé cosmétique selon la revendication 1, **caractérisé en ce qu'**il est effectué par administration quotidienne orale de la composition cosmétique.

3. Procédé cosmétique selon les revendications 1 ou 2, **caractérisé en ce qu'**il comprend une seule administration quotidienne de la composition cosmétique.

4. Procédé cosmétique selon l'une des revendications 1 à 3, dans lequel l'administration est répétée 2 à 3 fois par jour sur une journée.

5. Procédé cosmétique selon l'une des revendications 1 à 4, le procédé cosmétique étant appliqué sur une période prolongée d'au moins 30 jours ou de 4 à 15 semaines, avec éventuellement une ou plusieurs périodes d'interruption.

6. Procédé cosmétique selon l'une des revendications 1 à 5, **caractérisé en ce que** ledit isoflavonoïde est choisi parmi les isoflavones, les isoflavanones, les roténoïdes, les ptérocarpanes, les isoflavanes, les isoflavan-3-ènes, les 3-arylcoumarines, les 3-aryl-4-hydroxycoumarines, les coumestanes, les coumaronochromones, les α-méthyldésoxybenzoïnes et les 2-arylbenzofuranes.

7. Procédé cosmétique selon l'une des revendications 1 à 6, **caractérisé en ce que** ledit isoflavonoïde est choisi parmi la génistine, la daidzine, la génistéine, la daidzéine, la glycitine, l'equol, la formononétine, la cunéatine, l'isoprunétine et la prunétine, la cajanine, l'orobol, la pratenséine, le santal, la junipégénine A, la glycitéine, l'afrormosine, la rétusine, la tectorigénine, l'irisolidone, la jamaïcine et également les analogues et/ou les métabolites de ceux-ci.

8. Procédé cosmétique selon l'une quelconque des revendications 1 à 6, **caractérisé en ce que** ledit au moins un isoflavonoïde est une isoflavone ou un mélange d'isoflavones.

9. Procédé cosmétique selon la revendication 7, **caractérisé en ce que** ledit isoflavonoïde est choisi entre la daidzine, la daidzéine, la génistine et la génistéine ou un mélange de celles-ci.

10. Procédé cosmétique selon la revendication 8, **caractérisé en ce que** ladite au moins une isoflavone est un mélange d'isoflavones.

11. Procédé cosmétique selon la revendication 10, **caractérisé en ce que** ladite au moins une isoflavone est sous la forme d'un extrait d'isoflavones de soja.

12. Procédé cosmétique selon l'une des revendications 1 à 11, **caractérisé en ce que** la composition cosmétique est sous la forme d'unités galéniques choisies entre une solution buvable, un sirop, un comprimé, un comprimé enrobé, une gélule et une capsule.

13. Procédé cosmétique selon l'une des revendications 1 à 12, **caractérisé en ce que** les phytoestrogènes, de préférence les composés isoflavones, sont présents dans la composition cosmétique en une teneur allant de 0,0001 % à 50 % en poids, préférentiellement de 0,001 % à 30 % en poids et de préférence de 0,1 % à 10 % en poids par rapport au poids total de la composition cosmétique.

14. Procédé cosmétique selon l'une des revendications 1 à 13, **caractérisé en ce que** le caroténoïde, de préférence le lycopène, est présent dans la composition cosmétique en une teneur allant de 0,0001 % à 50 % en poids, préférentiellement de 0,001 % à 10 % en poids et de préférence même encore en une teneur allant de 0,02 % à 2 % en poids par rapport au poids total de la composition cosmétique.

15. Procédé cosmétique selon l'une des revendications 1 à 14, **caractérisé en ce que** la vitamine C est présente dans la composition cosmétique en une teneur allant de 0,0001 % à 50 % en poids, de préférence de 0,1 % à 30 % en poids et de préférence même encore en une teneur allant de 0,2 % à 12 % en poids par rapport au poids total de la composition cosmétique.

16. Procédé cosmétique selon l'une des revendications 1 à 15, **caractérisé en ce qu'**il comprend en outre l'administration combinée d'au moins une seconde composition destinée à avoir un effet bénéfique sur la peau.

17. Procédé cosmétique selon la revendication 16, dans lequel la seconde composition est administrée par voie orale ou par voie topique à la peau, en particulier par voie topique.

18. Procédé cosmétique selon la revendication 16, dans lequel la seconde composition comprend au moins un principe actif, en particulier un agent cosmétique, plus particulièrement un principe actif cosmétique qui est capable d'exercer un effet bénéfique sur l'hydratation de la peau.
